(19) **Europäisches Patentamt**
**European Patent Office**
**Office européen des brevets**

(11) **EP 4 016 048 B1**

(12) # EUROPEAN PATENT SPECIFICATION

(45) Date of publication and mention
of the grant of the patent:
**11.02.2026 Bulletin 2026/07**

(51) International Patent Classification (IPC):
***G01N 15/08*** (2006.01) ***G01N 15/02*** (2024.01)
***C06D 3/00*** (2006.01)

(21) Application number: **21214499.2**

(22) Date of filing: **14.12.2021**

(52) Cooperative Patent Classification (CPC):
**G01N 15/082; G01N 15/0255;** G01N 2015/0261;
G01N 2015/084

(54) **METHOD FOR TESTING BACTERIAL FILTRATION EFFICACY OF FABRICS**

VERFAHREN ZUR PRÜFUNG DER WIRKSAMKEIT DER BAKTERIELLEN FILTRATION VON GEWEBEN

PROCÉDÉ D'ESSAI DE L'EFFICACITÉ DE FILTRATION BACTÉRIENNE DE TISSUS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.12.2020 EP 20214273**

(43) Date of publication of application:
**22.06.2022 Bulletin 2022/25**

(73) Proprietor: **Sanko Tekstil Isletmeleri San. Ve Tic.
A.S.**
**16400 Inegol - Bursa (TR)**

(72) Inventors:
• **ÖZSEYHAN, Mehmet Erkan**
**16400 Inegol - Bursa (TR)**
• **AKBAS, Halil**
**16400 Inegol - Bursa (TR)**
• **KONUKOGLU, Fatih**
**16400 Turkey (TR)**
• **ÖZDEMIR, Mahmut**
**16400 Inegol - Bursa (TR)**
• **KILIÇKAN, Esin**
**16400 Inegol - Bursa (TR)**
• **KOCAMER, Adnan**
**16400 Inegol - Bursa (TR)**
• **ÖZKAN, Ahmet**
**16400 Inegol - Bursa (TR)**
• **YAYLA, Orhan**
**16400 Inegol - Bursa (TR)**

(74) Representative: **Gislon, Gabriele**
**Marietti, Gislon e Trupiano S.r.l.**
**Via Larga, 16**
**20122 Milano (IT)**

(56) References cited:
**JP-A- S62 149 331**

• **ANONYMOUS: "UNE EN 14683:2019+AC:2019 Medical face masks - Requirements and test methods", 31 December 2019, UNE EN NORM, EUROPEAN STANDARD, SPAIN, PAGE(S) 1 - 26, XP009527252**
• **COPLEY COPLEY: "Quality Solutions for Inhaler Testing", COPLEY SCIENTIFIC, 6 July 2015 (2015-07-06), pages 1 - 132, XP055326880, Retrieved from the Internet <URL:http://www. copleyscientific.com/files/ww/brochures/Inhaler %20Testing%20Brochure%202015_Rev4_Low% 20Res.pdf> [retrieved on 20161207]**
• **W.T. JENNY KWONG ET AL: "Comparison of Nebulized Particle Size Distribution with Malvern Laser Diffraction Analyzer Versus Andersen Cascade Impactor and Low-Flow Marple Personal Cascade Impactor", JOURNAL OF AEROSOL MEDICINE, vol. 13, no. 4, 27 March 2000 (2000-03-27), US, pages 303 - 314, XP055505571, ISSN: 0894-2684, DOI: 10.1089/ jam.2000.13.303**

**(Cont. next page)**

- LEHOFER BERNHARD ET AL: "Impact of atomization technique on the stability and transport efficiency of nebulized liposomes harboring different surface characteristics", EUROPEAN JOURNAL OF PHARMACEUTICS AND BIOPHARMACEUTICS, ELSEVIER SCIENCE PUBLISHERS B.V., AMSTERDAM, NL, vol. 88, no. 3, 23 October 2014 (2014-10-23), pages 1076 - 1085, XP029096793, ISSN: 0939-6411, DOI: 10.1016/J.EJPB.2014.10.009
- FARBER B I ET AL: "RESPIRATION FILTER PENETRATION USING SODIUM CHLORIDE AEROSOL", INFORMATION CIRCULAR - UNITED STATES, BUREAU OF MINES, US DEPT. OF THE INTERIOR, BUREAU OF MINES , WASHINGTON, DC, US, 30 June 1970 (1970-06-30), pages 1 - 10, XP001248176, ISSN: 1066-5544
- W.H  FINLAY: "Undersizing of droplets from a vented nebulizer caused by aerosol heating during transit through an anderson impactor", JOURNAL OF AEROSOL SCIENCE, 1 January 1999 (1999-01-01), AMSTERDAM, NL, pages 105 - 109, XP093160199, ISSN: 0021-8502, Retrieved from the Internet <URL:https://pdf. sciencedirectassets.com/271786/ 1-s2.0-S0021850200X00353/ 1-s2.0-S002185029800024X/main.pdf? X-Amz-Security-Token=IQoJb3JpZ2luX2VjEH waCXVzLWVhc3QtMSJGMEQCIGBsVLPpzCXtv c7wlWtUrrJMQy0JvPtvCwJ +Gr4Vp0HGAiAHcJrMoZkYxd9X+XTUx7GfxZ +jyT3hwzATcNcwgFJYrCq8BQjU////////// 8BEAUaDDA1OTAwMzU0Njg2NSIMUaZ4X> [retrieved on 20240507], DOI: 10.1016/ S0021-8502(98)00024-X

**Description**

Field of the invention

The present invention relates to a method of testing fabrics, in particular, fabrics that are suitable for the production of respiratory face masks. More specifically, the present invention relates to method for testing the bacterial filtration efficacy (BFE) of fabrics.

State of the art

Personal respiratory masks, also known as "face masks", "respiratory masks" or "filtering face masks", are protective devices used to protect the wearer's respiratory system from airborne particles. Facial masks are in fact worn over the nose and mouth of the user to protect him from unwanted material suspended in the air. In some embodiments (namely those without a valve that let breath exit the mask) the mask also acts as a filter to prevent or reduce the leakage of any particles suspended in the user's breath to protect other people from possible infections of the person wearing mask. Known masks are typically made of non-woven fabric in two forms: a cup-shaped shape or a flat shape in which the non-woven fabric is partially folded on itself to be able to adapt to the shape of the face when worn. One type of flat mask is known as a "surgical mask". Flat masks made of woven fabric are also known.

A mask requires the presence of straps or bands, preferably of elastic material, which generally are in the form of loops that pass around the user's ears or around the user's head to keep the mask in the desired position on the user's face. The straps or equivalent retaining means are typically made separately and are attached to the body of the mask, by means such as sewing, gluing, ultrasonic welding, stapling or other means commonly known to those skilled in the art. Protective devices are also known in which the retaining means are loops of elastic material attached to a folded portion of the mask body.

A face mask also typically requires the presence of a strip of plastically bendable material, generally a metal strip, that is located at the upper edge of the mask, i.e., at the side of the mask that is transversally floating over the bridge of the nose of the user when the mask is worn. This so-called "nose clip" may actually be made of any material provided it can be easily bended in a shape fitting to the bridge of the nose of the user to improve the air tightness of the mask.

In order to test the filtration efficacy of masks, the standard method according to EN 14683:2019+AC:2019 (EN 14683) is generally applied. According to EN 14683, bacterial filtration efficacy of masks is tested using a six-stage cascade impactor, and an aerosol chamber. A sample of the mask material is clamped between a six-stage cascade impactor and an aerosol chamber. An aerosol of *Staphylococcus aureus (ATCC* 6538) is produced by a nebulizer, introduced into the aerosol chamber, and drawn through the mask material and the impactor under vacuum. The bacterial filtration efficiency (BFE) of the mask is given by the number of colony forming units passing through the sample of mask material expressed as a percentage of the number of colony forming units present in the challenge aerosol. EN 14683 requires that the mean particle size (MPS) of the bacterial challenge, i.e., the mean particle size of the droplets containing the bacteria, when in contact with the cascade impactor, is maintained between 2.7 and 3.3 $\mu$m. The mean particle size of the droplets containing bacteria directly influences the data that are used to calculate the bacterial filtration efficiency of the mask. Therefore, a mean particle size that does not fall within the required range would provide a not reliable evaluation of the bacterial filtration efficiency of the mask.

Several problems are connected to EN 14683. One problem is that, whilst EN 14683 is used for testing masks made of woven fabrics, the same fabric may give different results from a test to another. Additionally, it is difficult to maintain the MPS of droplets containing bacteria in the required range when carrying out tests according EN 14683. A MPS lower than the required MPS (for example, in the range between 1.5 $\mu$m and 2.3 $\mu$m), will not satisfy the requirements of the standard EN 14683. Moreover, in view of the problem of maintaining the mean particle size of the droplets, also the formation of individual bacterial colonies on the petri dishes of the impactor and, as a consequence, the count of the colonies, results to be difficult in the MPS is not the required one.

Summary of the invention

An aim of the present invention is to solve the above discussed problems and to provide a method for determining bacterial filtration efficiency of fabrics in a reliable way.

Another aim of the present invention is to provide a method for determining bacterial filtration efficiency of fabrics which allows to maintain the mean particle size of the droplets in the required range.

A further aim of the present invention is to provide a method for determining bacterial filtration efficiency of fabrics which is suitable to test woven fabrics, e.g., respiratory masks made of woven fabrics.

These and other aims are reached by the present invention that relates to a method for testing the bacterial filtration efficiency of a fabric, according to claim 1. Preferred embodiments of the invention are object of the dependent claims.

The present invention refers to a method of testing the bacterial filtering efficiency of a fabric according to claim 1. The method includes the steps of preparing a solution including bacteria and peptone water comprising NaCl, feeding said solution including bacteria to a nebulizer, generating an aerosol of said bacterial solution and flowing said solution through a cascade impactor to provide a plurality of bacteria colonies in a plurality of plates present in a plurality of stages of said cascade impactor, characterized in that the concentration of NaCl in the peptone water used to prepare said bacterial solution is in the range of 30g/L to 150g/L and in that the temperature of said cascade impactor is in the range of -15°C to 15°C.

In embodiments, the method is carried out according to EN 14683:2019+AC:2019 E modified to provide an NaCl concentration of the peptone water and a refrigeration of the cascade impactor as recited above.

In embodiments, the NaCl concentration is in the range of 60g/L to 150g/L, preferably in the range of 60g/L to 120g/L, more preferably of 100g/L.

In embodiments, the temperature of the cascade impactor is in the range of -4°C to 15°C, preferably in the range of 0°C to 12°C.

In the testing conditions, the nebulizer is capable to generate an aerosol of a saline solution, free from bacteria, having Mean Particle Size in the range of 2.7 to 3.3 $\mu$m.

In embodiments, the nebulizer is selected from a jet nebulizer, an ultrasonic nebulizer, and a mesh nebulizer.

The present description further refers to a method for bringing into a required size range the Mean Particle Size of particles generated by a nebulizer in a device for testing Bacterial Filtration Efficiency of a fabric, the device comprising a nebulizer for generating an aerosol of a bacterial solution and a cascade impactor including a plurality of stages, said method comprising the steps of: generating an aerosol of a solution including bacteria, peptone water an NaCl; flowing said aerosol through the cascade impactor to provide a plurality of bacteria colonies in a plurality of plates present in the plurality of stages of said cascade impactor, characterized in comprising the steps of: performing a positive control run by feeding said aerosol to said cascade impactor; determining the Mean Particle Size of the aerosol particles based on the resulting number of bacterial colonies; comparing the obtained Mean Particle Size with a required range of Mean Particle Size; modifying the control run conditions by increasing or decreasing the concentration of NaCl in said bacterial solution and/or the temperature of said cascade impactor; performing at least another positive control run under said modified conditions and repeating said steps until the resulting Mean Particle Size is within the required range.

The mentioned required range of the Mean Particle Size may be from 2.7 $\mu$m to 3.3 $\mu$m.

The method for bringing into a required size range the Mean Particle Size of particles generated by a nebulizer in a device for testing Bacterial Filtration Efficiency of a fabric may be carried out according to EN 14683:2019+AC:2019 E, optionally the run conditions being modified by increasing or decreasing the concentration of NaCl in said bacterial solution and/or the temperature of said cascade impactor.

In embodiments, bacteria are selected from the group consisting of Mycobacterium tuberculosis, Streptococcus pneumoniae, Legionella pneumophilla, Staphylococcus aureus, Bacillus subtilis, and Escherichia coli, preferably is Staphylococcus aureus, more preferably is Staphylococcus aureus ATCC 6538.

The fabric sample is placed between the first stage and the inlet cone of said cascade impactor.

The present description further refers to a device for testing Bacterial Filtration Efficiency of a fabric, the device comprising a nebulizer for generating an aerosol of a bacterial solution and a cascade impactor including a plurality of stages, characterized in comprising refrigerating means to refrigerate the cascade impactor.

The device may be a device according to EN 14683:2019+AC:2019 E, preferably provided with refrigerating means to refrigerate the cascade impactor.

In embodiments, the refrigerating means are provided at the exit portion of said cascade impactor.

Detailed Disclosure of the invention

The present invention relates to a method of testing the bacterial filtering efficiency of a fabric, the method including the steps of preparing a solution including bacteria, peptone water and NaCl, feeding the solution including bacteria to a nebulizer, generating an aerosol of said bacterial solution and flowing said solution through a cascade impactor to provide a plurality of bacteria colonies in a plurality of plates present in a plurality of stages of said cascade impactor, characterized in that the concentration of NaCl in the peptone water used to prepare said bacterial solution is in the range of 30g/L to 150g/L, preferably in the range of 60g/L to 120g/L, and in that the temperature of said cascade impactor is in the range of -15°C to 15°C, preferably in the range of -4°C to 15°C, and more preferably in the range 0°C to 12°C .

As used herein, the term "bacterial filtration efficacy" or "BFE" refers to the efficiency of a face mask, e.g., the fabric forming the face mask, to act as a barrier to bacterial penetration. According to EN 14683, bacterial filtration efficacy must be ≥%95 for type I masks, and ≥%98 for type II / type IIR masks.

According to the invention, bacteria are diluted in a solution including peptone water and NaCl, wherein the concentration of NaCl in the peptone water used to prepare the bacterial solution is in the range of 30g/L to 150g/L, preferably is in the is in the range of 60g/L to 150g/L, more preferably is in the range of 60g/L to 120g/L.

According to the invention, the temperature of said cascade impactor is in the range of -15°C to 15°C, preferably in the range of -4°C to 15°C, more preferably in the range of 0°C to 12°C. In embodiments, the temperature of the cascade impactor may be in the range of 5°C to 15°C.

It has been observed that by using a concentration of NaCl between 30 g/L and 150 g/L in the peptone water, and at the same time, using a cascade impactor at a temperature in the range from -15°C to 15°C, it is possible to obtain particles having MPS in the range from 2.7 $\mu$m to 3.3 $\mu$m in a reproducible and reliable way, thus allowing a correct evaluation of the amount of the bacteria that are blocked by the fabric, i.e., by the mask. Additionally, it has been observed that only the combined use of the mentioned concentration of NaCl in the peptone water and the impactor cooled at the mentioned temperature allows to obtain the required MPS, i.e., in the range from 2.7 $\mu$m to 3.3 $\mu$m. In fact, it has been observed that, when a peptone water comprising NaCl in an amount between 30 g/L and 150 g/L is used, without cooling the cascade impactor, the obtained MPS is in the range from 2.4 $\mu$m to 2.7 $\mu$m, which is in general not acceptable, apart from those cases in which the obtained MPS is exactly 2.7 $\mu$m. Therefore, it has been observed that the use of a peptone water comprising NaCl in an amount between 30 g/L and 150 g/L, without cooling the cascade impactor provides for results that are not sufficiently reliable. It was also observed that, when a cooled cascade impactor is used, using the known NaCl concentration in the peptone water, the obtained MPS is in the range from 2.4 $\mu$m to 2.6 $\mu$m, which is not acceptable.

According to embodiments, the method is a method of testing the bacterial filtering efficiency of a woven fabric.

According to embodiments, the aerosol of bacterial solution is generated inside an aerosol chamber, which is in communication with the cascade impactor.

According to embodiments, the method of the invention is suitable to test BFE with respect to different bacterial. According to embodiments, bacteria may be selected from airborne bacteria (i.e., bacteria present in the atmosphere). According to embodiments, the bacteria may be selected from the group consisting of *Mycobacterium tuberculosis, Streptococcus pneumoniae, Legionella pneumophilla, Staphylococcus Aureus, Bacillus Subtilis,* and *Escherichia Coli.* According to preferred embodiments, bacteria are *Staphylococcus aureus,* preferably *Staphylococcus aureus ATCC 6538.*

According to embodiments, the method is carried out according to EN 14683:2019+AC:2019 E modified to provide an NaCl concentration of the peptone water and a refrigeration of the cascade impactor as mentioned above. According to embodiments, the method is carried out according to EN 14683:2019+AC:2019 E modified to test bacteria different from *Staphylococcus aureus.* For example, the method is carried out according to EN 14683:2019+AC:2019 E modified to test bacteria be selected from the group consisting of *Mycobacterium tuberculosis, Streptococcus pneumoniae, Legionella pneumophilla, Bacillus Subtilis,* and *Escherichia Coli,* in addition to *Staphylococcus Aureus.* According to embodiments, the method is carried out according to EN 14683:2019+AC:2019 E modified to provide an NaCl concentration of the peptone water and a refrigeration of the cascade impactor as mentioned above, as well as to use bacteria that are optionally different from *Staphylococcus aureus.*

In embodiments of the method of the invention, the bacterial solution is delivered to the nebulizer, introduced into an aerosol chamber, and drawn through the impactor under vacuum.

The process for testing bacterial filtration efficacy of a fabric may be carried out, in general, as follows. A first positive control, without fabric samples, is carried out. The petri dishes of the impactor are removed and replaced with fresh plates. The fabric sample is placed between the first stage and the inlet cone of the cascade impactor. After all the samples to be tested have been challenged, a further positive control without fabric is carried out. A negative control is also carried out, without fabric, by passing air, without addition of the bacterial challenge, through the cascade impactor. All the plates obtained are incubated, preferably between 35°C and 39°C, for a time in the range from 20 to 52 h. The Mean Particle Size and Bacterial filtration Efficacy are then determined for each sample of fabric.

In embodiments, for each sample and control run, the number of colonies on each plate are counted and added up to give the total number of CFU collected by the cascade impactor. The "positive hole" conversion table in accordance with the instructions of the cascade impactor manufacturer is used to correct the count for stages 3 to 6. For example, as the cascade impactor is an Andersen impactor, "Table 1: Positive Hole Conversion Table", reported in the paper of Ariel A. Andersen, "NEW SAMPLER FOR THE COLLECTION, SIZING, AND ENUMERATION OF VIABLE AIRBORNE PARTICLES", J Bacteriol. 1958 Nov; 76(5): 471-484, at page 474 is used.

For the two positive control runs, the mean of the two totals is calculated, to obtain the "average converted positive control", as indicated in the formula below.

From the positive control plates the mean particle size (MPS) of bacterial solution aerosol is calculated.

According to embodiments of the method of the invention, the MPS is determined by counting the colonies of the two positive controls, as above discussed. The counted numbers (converted using the positive hole conversion table where applicable) obtained for each plate are multiplied by the hole size coefficient of the cascade impactor, which is different for each stage (e.g., 7 for stage (plate) 1, 4.7 for stage (plate) 2, 3.3 for stage (plate) 3, 2.1 for stage (plate) 4, 1.1 for stage (plate) 5 and 0.65 for stage (plate) 6). The multiplied numbers of each plate are summed, for each positive control, to obtain the total multiplied converted count of the positive controls. Such total multiplied converted count of the positive controls, obtained for each positive control, is divided by the total count of the respective positive control obtained before the multiplication by the hole size coefficient, to obtain the MPS for each positive control run. The mean value of the two MPS values so obtained is the MPS of the particles of the bacterial solution aerosol.

According to embodiments, the Bacterial Filtration Efficiency is then calculated.

The colonies of each plate obtained from a fabric sample are counted (and converted using the positive hole conversion table where applicable) and summed to obtain a "converted mask value", as indicated in the formula below. The difference between the average converted positive control count (i.e., the "average converted positive control", mentioned above) and the total converted count of the fabric, i.e., the "converted mask value" mentioned above, is divided by the "average converted positive control". The obtained result is multiplied by 100 to obtain the BFE expressed as percentage of the number of colony-forming units present in the bacterial challenge aerosol.

According to embodiments, the bacterial filtration efficiency is calculated by the following formula:

$$Bacterial\ Filtration\ Efficiency\ (BFE)$$
$$= \frac{Average\ converted\ positive\ control - Converted\ Mask\ Value}{Average\ converted\ positive\ control} \ x\ 100$$

As above mentioned, the method of the invention is characterized in that the concentration of NaCl in the peptone water used to prepare the bacterial solution is in the range of 30g/L to 150g/L and in that the temperature of the cascade impactor is in the range of -15°C to 15°C.

According to embodiments, the NaCl concentration is in the range of 60g/L to 150g/L, preferably in the range of 60g/L to 120g/L, and more preferably of 100g/L.

According to embodiments, the temperature of the cascade impactor is in the range of -4°C to 15°C, preferably of 0°C to 12°C.

According to embodiments, the temperature of the cascade impactor may be regulated by incubating the cascade impactor in a refrigerator. For example, the cascade impactor may be incubated in a -80°C refrigerator for at least 5 minutes, or -20°C for at least 15 minutes, or +4°C for at least 1 hour.

In the testing conditions the nebulizer is capable to generate an aerosol of a saline solution, free from bacteria, having Mean Particle Size of $3.0 \pm 0.3\ \mu m$. For example, saline solutions may be NaCl or NaF (sodium fluoride) solutions.

Examples of suitable nebulizers are jet, ultrasonic and mesh nebulizers. An exemplary suitable nebulizer is the nebulizer OMRON HEALTHCARE Co., Ltd. (Japan), model: NE-C28P-E. This is a jet nebulizer, which provides for a nebulization rate of 0.05 ml/min, and an aerosol mean particle size of $3\ \mu m$, measured using a saline solution, free from bacteria.

The cascade impactor is an Andersen six-stages cascade impactor. The Andersen six-stage cascade impactor, disclosed in Ariel A. Andersen, "NEW SAMPLER FOR THE COLLECTION, SIZING, AND ENUMERATION OF VIABLE AIRBORNE PARTICLES", J Bacteriol. 1958 Nov; 76(5): 471-484. Each one of the six stages of the impactor includes a petri dish.

The present description also refers to a method for bringing into a required size range the Mean Particle Size of particles generated by a nebulizer in a device for testing Bacterial Filtration Efficiency of a fabric, the device comprising a nebulizer for generating an aerosol of a bacterial solution and a cascade impactor including a plurality of stages, said method comprising the steps of: generating an aerosol of a solution including bacteria, peptone water an NaCl; flowing said aerosol through the cascade impactor to provide a plurality of bacteria colonies in a plurality of plates present in a plurality of stages of the cascade impactor, characterized in comprising the steps of: performing at least one positive control run by feeding said aerosol to the cascade impactor; determining the Mean Particle Size of the aerosol particles based on the resulting number of bacterial colonies; comparing the obtained Mean Particle Size with a required range of MPS; modifying the control run conditions by increasing or decreasing the concentration of NaCl in said bacterial solution and/or the temperature of the said cascade impactor; performing at least another positive control run under said modified conditions and repeating said steps until the resulting Mean Particle Size, i.e., a obtained value of Mean Particle Size, is within the required range. According to embodiments, the required range for the Mean Particle Size is from 2.7 $\mu m$ to 3.3 $\mu m$.

The method for bringing into a required size range the Mean Particle Size of particles generated by a nebulizer in a device for testing Bacterial Filtration Efficiency of a fabric may be carried out according to EN 14683:2019+AC:2019 E. When the method for bringing into a required size range the Mean Particle Size of particles generated by a nebulizer in a device for testing Bacterial Filtration Efficiency of a fabric is carried out according to EN 14683:2019+AC:2019 E, run conditions are optionally modified by increasing or decreasing the concentration of NaCl in the bacterial solution and/or by increasing or decreasing the temperature of the cascade impactor. The method may be carried out according to EN 14683:2019+AC:2019 E modified, optionally, also to test bacteria different from *Staphylococcus aureus,* for example, using bacteria be selected from the group consisting of *Mycobacterium tuberculosis, Streptococcus pneumoniae, Legionella pneumophilla, Bacillus Subtilis,* and *Escherichia Coli,* in addition to *Staphylococcus Aureus.* The method may be carried out according to EN 14683:2019+AC:2019 E modified to increase or decrease the NaCl concentration in the bacterial solution and/or the temperature of said cascade impactor, and/or to use bacteria that are optionally different from *Staphylococcus aureus.*

The bacteria may be *Staphylococcus aureus,* preferably *Staphylococcus aureus* AATC 6538.

The present description also refers to a device for testing Bacterial Filtration Efficiency of a fabric, the device comprising a nebulizer for generating an aerosol of a bacterial solution and a cascade impactor including a plurality of stages, characterized in comprising refrigerating means to refrigerate the cascade impactor.

The device may be a device according to EN 14683:2019+AC:2019 E. In other words, a device according to EN 14683:2019+AC:2019 E may be provided with refrigerating means to refrigerate the cascade impactor. For example, the refrigerating means may be a refrigerating plate or container onto or into which the cascade impactor is placed.

The refrigerating means may be provided at the exit portion of the cascade impactor.

Examples

The following examples relate to exemplary embodiments of the present invention and are to be considered as illustrative and non-limiting with respect of the scope of the invention.

Example 1- Reagents and materials

*Tryptic soy agar (TSA)*

Tryptic soy agar is a solid culture medium. The composition is reported in Table 1, with reference to the liquid solution used to produce the solid medium.

| Table 1 (formula/litre) | |
|---|---|
| Enzymatic Digest of Casein | 15 g |
| Enzymatic Digest of Soybean Meal | 5 g |
| Sodium Chloride | 5 g |
| Agar | 15 g |
| Distilled water | 1000 ml |
| Final pH | 7.3 $\pm$ 0,2 at 25°C |

*Tryptic Soy Broth (TSB)*

Tryptic soy broth is a liquid culture medium. The composition is reported in Table 2.

| Table 2 (formula/litre) | |
|---|---|
| Enzymatic Digest of Casein | 17 g |
| Enzymatic Digest of Soybean Meal | 3 g |
| Sodium Chloride | 5 g |
| Dipotassium phosphate | 2.5 g |
| Dextrose | 2.5 g |
| Distilled water | 1000 ml |
| Final pH | 7.3 $\pm$ 0,2 at 25°C |

*Peptone water - according to standard method EN 14683*

Peptone water is a liquid medium used for diluting the bacterial culture before the test according to standard method EN 14683. The composition is reported in Table 3.

| Table 3 (formula/litre) | |
|---|---|
| Peptone | 10 g |
| Sodium Chloride (NaCl) | 5 g |
| Distilled water | 1000 ml |
| Final pH | 7,2 ± 0,2 at 25°C |

*Peptone water - according to the invention*

Peptone water according to the invention is a liquid medium used for diluting the bacterial culture before the test according to the invention. The composition of an exemplary embodiment is reported in Table 4.

| Table 4 (formula/litre) | |
|---|---|
| Peptone | 10 g |
| Sodium Chloride (NaCl) | 100 g |
| Distilled water | 1000 ml |
| Final pH | 7,2 ± 0,2 at 25°C |

Example 2- exemplary procedure

According to the invention, the cascade impactor is cooled to a temperature in the range of -15°C to 15°C, for example, by keeping the impactor for at least 5 minutes at -80°C in a refrigerator.

Additionally, according to the invention, peptone water includes NaCl at a concentration of in the range of 30g/L to 150g/L.

**1) Preparation of media**

    a. Liquid media

        i. Tryptic Soy Broth (TSB)
        ii. Peptone Water

    b. Solid medium

        i. Tryptic Soy Agar (TSA)

**2) Bacterial growth**

    a. Resuscitation of *Staphylococcus aureus* bacteria

        i. Growth of *S. aureus* in TSB medium at 37°C overnight.

    b. Inoculation of *Staphylococcus aureus* bacteria using streak plate method

        i. Growth of *S. aureus* in TSA medium at 37°C overnight.

**3) Test phase**

a. Conditioning of test fabric

i. Hanging the test fabric in the climate chamber which provides constant temperature (21 ± 5°C) and relative humidity (85 ± 5%) for at least 4 hours.

b. Inoculation of *Staphylococcus aureus* bacteria in TSB medium

i. Selecting a single colony of *S. aureus* bacteria using inoculation loop, adding that colony in TSB medium, and growing bacteria at 37°C for 4 hours.

c. Dilution of *Staphylococcus aureus* bacteria

i. Confirmation of adequate growth of bacteria by measuring turbidity of bacterial solution using McFarland densitometer and/or OD600 spectrophotometer.
ii. Diluting bacterial solution with peptone water to obtain $5 \times 10^5$ colony forming unit (cfu).

d. Test operation
i. Calibrating Bacterial Filtration Efficiency (BFE) device by setting vacuum flow rate to 28.3 L/min (1 cubic foot per minute) and running for 2 minutes (1 minute for bacterial challenge and the other one minute for maintaining the airflow without running the nebulizer).
ii. Order of bacterial filtration efficiency tests is as follows (Table 5):

| Table 5 - ORDER OF BACTERIAL FILTRATION EFFICIENCY TEST | | | | | | | |
|---|---|---|---|---|---|---|---|
| 1 | 2 | 3 | 4 | 5 | 6 | 7 | 8 |
| Positive control 1 | Mask 1 | Mask 2 | Mask 3 | Mask 4 | Mask 5 | Positive control 2 | Negative control |

iii. Numbering TSA petri plates from "Control 1-1" to "Control 1-6" with permanent pen, then locating them into Andersen Cascade Impactor (ACI) accordingly.
iv. Pouring bacterial solution into nebulizer chamber.
v. Running the test without any test fabric for 2 minutes to obtain positive control.
vi. Removing TSA petri plates from ACI.
vii. Taking the test fabric (i.e., the test mask) from climate chamber.
viii. Removing ear loop and cutting two sides to open the pleated part of test fabric (i.e., the test mask). The test area shall be minimum 49 cm$^2$ .
ix. Numbering TSA petri plates from "Mask 1-1" to "Mask 1-6" with permanent pen, then locating them into Andersen Cascade Impactor (ACI) accordingly.
x. Locating test fabric to the top of ACI, and clamping tightly to prevent any leakage.
xi. Running the test with test fabric for 2 minutes (1 minute for bacterial challenge and the other one minute for maintaining the airflow without running the nebulizer) to obtain mask 1 result.
xii. Repeat the same protocol until 5th mask.
xiii. Running the second positive control without any fabric (mask).
xiv. Cleaning the nebulizer from bacterial solution, then running for negative control without mask and bacteria.
e. Growth of *Staphylococcus aureus* bacteria in incubator

i. Locating all petri plates (8 set x 6 plates = 48 plates) at incubator at 37°C±2°C for between 20 hours and 52 hours.

**4) Preparation of Report**

a. Counting of *Staphylococcus aureus* bacteria
i. Place TSA petri plate on a black surface, then starting to count by permanent marker or Promega Colony Count app depending on the urgency.
ii. Write down all bacteria number on a paper report.
iii. Transfer all data to Excel, then convert all data except petri 1 and petri 2 according to the positive hole conversion table from the cascade impactor manual. In the present example, an Andersen cascade impactor and the related "Table 1: Positive Hole Conversion Table", reported in the paper of Ariel A. Andersen, "NEW SAMPLER FOR THE COLLECTION, SIZING, AND ENUMERATION OF VIABLE AIRBORNE PARTICLES", J Bacteriol. 1958 Nov; 76(5): 471-484, at page 474 were used. In the mentioned table, the Actual bacteria number is represented as "r", converted bacteria number is represented as "P". For example, if actual bacteria number is 121, then converted bacteria number is 144.

iv. Numbers in italics represents no conversion, underlines represent summation of converted positive control.

| Table 6 - CONVERSION OF ACTUAL POSITIVE CONTROL USING "POSITIVE HOLE CONVERSION TABLE" | | | | | | | |
|---|---|---|---|---|---|---|---|
| | Petri 1 (CFU/plate) | Petri 2 (CFU/plate) | Petri 3 (CFU/plate) | Petri 4 (CFU/plate) | Petri 5 (CFU/plate) | Petri 6 (CFU/plate) | Total CFU |
| **Actual Positive Control 1** | *124* | *210* | 251 | 312 | 283 | 9 | 1189 |
| **Actual Positive Control 2** | *134* | *293* | 332 | 366 | 280 | 15 | 1420 |
| **Converted Positive Control 1** | *124* | 210 | 395 | 606 | 492 | 9 | <u>1836</u> |
| **Converted Positive Control 2** | *134* | 293 | 709 | 986 | 482 | 15 | <u>2619</u> |
| **Average Converted Positive Control** | 129 | 251.5 | 552 | 796 | 487 | 12 | <u>2227.5</u> |

v. Calculation of mean total bacteria number

1. Summation of converted positive control 1.

2. Summation of converted positive control 2.

3. Calculate the average value of two converted positive controls.

4. According to EN14683 Standard, mean value of two converted positive control must be between 1700 and 3000 cfu.

| Table 7 - CONVERSION OF ACTUAL POSITIVE CONTROL USING "POSITIVE HOLE CONVERSION TABLE" | | | | | | | |
|---|---|---|---|---|---|---|---|
| | Petri 1 (CFU/plate) | Petri 2 (CFU/plate) | Petri 3 (CFU/plate) | Petri 4 (CFU/plate) | Petri 5 (CFU/plate) | Petri 6 (CFU/plate) | Total CFU |
| **Actual Positive Control 1** | *124* | *210* | 251 | 312 | 283 | 9 | 1189 |
| **Converted Positive Control 1** | *124* | *210* | 395 | 606 | 492 | 9 | <u>1836</u> |
| **Hole Size coefficient** | 7 | 4.7 | 3.3 | 2.1 | 1.1 | 0.65 | |
| **Multiplied converted positive control 1** | 868 | 987 | 1303.5 | 1272.6 | 541.2 | 5.9 | **4978.2** |

$$Mean\ particle\ size\ 1\ (MPS\ 1) = \frac{4978.2}{1836} = 2.71$$

**Table 8 - CONVERSION OF ACTUAL POSITIVE CONTROL USING "POSITIVE HOLE CONVERSION TABLE"**

| | Petri 1 (CFU/plate) | Petri 2 (CFU/plate) | Petri 3 (CFU/plate) | Petri 4 (CFU/plate) | Petri 5 (CFU/plate) | Petri 6 (CFU/plate) | Total CFU |
|---|---|---|---|---|---|---|---|
| Actual Positive Control 2 | 134 | 293 | 332 | 366 | 280 | 15 | 1420 |
| Converted Positive Control 2 | 134 | 293 | 709 | 986 | 482 | 15 | 2619 |
| Hole Size coefficient | 7 | 4.7 | 3.3 | 2.1 | 1.1 | 0.65 | |
| Multiplied converted positive control 2 | 938 | 1377.1 | 2339.7 | 2070.6 | 530.2 | 9.8 | **7265,4** |

$$Mean\ particle\ size\ 2\ (MPS\ 2) = \frac{7265,4}{2619} = 2.77$$

$$Average\ Mean\ particle\ size\ (MPS) = \frac{2.71 + 2.77}{2} = 2.74$$

vi. Calculation of mean particle size
1. Convert actual bacteria number of two positive controls using the Positive Hole Conversion Table (in the present example, the "Table 1: Positive Hole Conversion Table", reported in the paper of Ariel A. Andersen, mentioned above, was used).
2. Multiply converted bacteria numbers from positive control plate 1 to 6 with the respective hole size coefficient, i.e., in this case, 7, 4.7, 3.3, 2.1, 1.1, and 0.65 respectively.
3. Summation of multiplied numbers for positive control 1
4. Summation of multiplied numbers for positive control 2
5. Divide total value of the two multiplied numbers of the two control runs with the total value of the two converted positive controls, to obtain MPS for each positive control.
6. calculate the Mean value of MPS between the two MPS for positive controls.
7. According to 14683 Standard, mean particle size must be between 2,7 μm and 3.3 μm.

**Table 9 - CONVERSION OF ACTUAL MASK VALUE USING "POSITIVE HOLE CONVERSION TABLE"**

| | Petri 1 (CFU/plate) | Petri 2 (CFU/plate) | Petri 3 (CFU/plate) | Petri 4 (CFU/plate) | Petri 5 (CFU/plate) | Petri 6 (CFU/plate) | Total CFU |
|---|---|---|---|---|---|---|---|
| Actual Mask Value | 1 | 1 | 15 | 13 | 51 | 15 | 96 |
| Converted Mask Value | 1 | 1 | 15 | 13 | 55 | 15 | 100 |

$$Bacterial\ Filtration\ Efficiency\ (BFE)$$
$$= \frac{Average\ converted\ positive\ control - Converted\ Mask\ Value}{Average\ converted\ positive\ control} \times 100$$

$$Bacterial\ Filtration\ Efficiency\ (BFE) = \frac{2227.5 - 100}{2227.5}\ x\ 100 = 95.51\%$$

vii. Calculation of bacterial filtration efficiency

1. write down all bacteria number on a paper report

2. transfer all data to Excel, then convert all data except petri 1 and petri 2 according to "Table 1: positive Hole Conversion Table", as above mentioned

3. Use converted mask data and average converted positive control for % BFE calculation for each fabric (mask) tested (an example of fabric data for one mask is reported in Table 9, wherein numbers in italics represents no conversion, underlines represent summation of converted fabric values). In order to obtain the final BFE result, 5 mask (i.e., 5 fabric samples) should be tested and averaged.

4. According to EN 14683 Standard, bacterial filtration efficacy must be ≥%95 for type I masks, and ≥%98 for type II / type IIR masks.

Example 3- comparison between EN 14683 and the method of the invention

In the present example, several samples of the same woven fabric were tested.

In the test carried out according to the invention, the same conditions of the test carried out according to EN 14683 were used, except for concentration of NaCl in peptone water and for the temperature of the impactor, that were according to the method of the invention.

BFE Test with EN 14683

All the solutions and conditions were according to EN 14683. It was found that average mean particle size is 1.81 μm and average BFE result is 78,15 %.

| Table 10 - Total and converted cfu values | | | | | | | | |
|---|---|---|---|---|---|---|---|---|
| | Petri 1 | Petri 2 | Petri 3 | Petri 4 | Petri 5 | Petri 6 | Total cfu | Converted total (cfu) |
| Positive control 1 | 48 | 43 | 262 | 355 | 366 | 254 | 1328 | 2780 |
| Sample 1 | 1 | 6 | 8 | 44 | 198 | 175 | 432 | 565 |
| Sample 2 | 3 | 8 | 9 | 52 | 201 | 183 | 456 | 600 |
| Sample 3 | 2 | 5 | 7 | 33 | 191 | 188 | 426 | 562 |
| Sample 4 | 1 | 2 | 7 | 35 | 177 | 190 | 412 | 539 |
| Sample 5 | 0 | 1 | 5 | 30 | 169 | 193 | 398 | 520 |
| Positive control 2 | 31 | 41 | 200 | 338 | 357 | 225 | 1192 | 2318 |

| Table 11 - Average Mean particle size and Converted total bacteria. | | | |
|---|---|---|---|
| | Positive control 1 | Positive control 2 | Average |
| Converted total bacteria (cfu) | 2780 | 2318 | 2549 |
| Mean particle size (μm) | 1,84 | 1,76 | 1,81 |

| Table 12 - Bacteria Filtration Efficiency (%) according to EN 14683 method | |
|---|---|
| Sample 1 | 77,83 |
| Sample 2 | 76,46 |
| Sample 3 | 77,95 |
| Sample 4 | 78,85 |
| Sample 5 | 79,60 |

(continued)

| Table 12 - Bacteria Filtration Efficiency (%) according to EN 14683 method | |
|---|---|
| Average | 78,14 |

*BFE Trial with the method of the invention*

As above mentioned, the method of the invention allows to maintain the mean particle size in the range between 2.7 $\mu$m and 3.3 $\mu$m. The method of the invention is different from EN14683 standard, in particular, in the percentage (%) of NaCl in the Peptone water, and in the Andersen cascade temperature.

In the present example:

**Peptone Water Preparation:** Peptone water according to the invention, having the composition disclosed in Table 4, here above reported was used. In particular, a commercial peptone water product (distributed by Condalab) was used. According to the formulation of such product, 1.5 grams of powder contain 0.5 gram of NaCl and 1 gram of peptone. 9.5 grams of NaCl were added into the solution to make a 10 gram / 100 ml NaCl solution which has a final concentration of 10% NaCl. Solution was autoclaved at 121 °C for 15 minutes.

**Andersen Cascade Impactor Preparation:** Six stages Andersen Cascade was cooled, in the present example, on the -80°C refrigerator for 5 minutes.

| Table 13 - total and converted CFU values | | | | | | | | |
|---|---|---|---|---|---|---|---|---|
| | Petri 1 | Petri 2 | Petri 3 | Petri 4 | Petri 5 | Petri 6 | Total cfu | Converted total (cfu) |
| **Positive control 1** | 120 | 235 | 314 | 272 | 292 | 11 | 1244 | 1961 |
| **Sample 1** | 0 | 3 | 10 | 93 | 72 | 0 | 178 | 198 |
| **Sample 2** | 10 | 9 | 75 | 149 | 3 | 0 | 246 | 291 |
| **Sample 3** | 2 | 1 | 18 | 83 | 41 | 0 | 145 | 157 |
| **Sample 4** | 13 | 1 | 26 | 49 | 83 | 0 | 172 | 186 |
| **Sample 5** | 1 | 2 | 37 | 71 | 82 | 2 | 195 | 214 |
| **Positive control 2** | 201 | 211 | 375 | 302 | 156 | 4 | 1249 | 2286 |

| Table 14 - Average Mean particle size and Converted total bacteria. | | | |
|---|---|---|---|
| | Positive control 1 | Positive control 2 | Average |
| Converted total bacteria (cfu) | 1961 | 2286 | 2123.5 |
| Mean particle size ($\mu$m) | 2,81 | 3.26 | 3.06 |

| Table 15 - Bacteria Filtration Efficiency (%) according to the method of the invention | |
|---|---|
| Sample 1 | 90.68 |
| Sample 2 | 86.30 |
| Sample 3 | 92.61 |
| Sample 4 | 91.24 |
| Sample 5 | 89.92 |
| **Average** | **90.15** |

It was found that average mean particle size is 3.06 $\mu$m and average BFE result is 90,15 %.

**Conclusion**

A 12 % BFE result differences between EN 14683 standard and the method of the invention was observed.

| Table 16 - Comparison of result of EN 14683 and the method of the invention. | | |
|---|---|---|
| | EN 14683 | Method of the invention |
| Sample 1 | 77,83 | 90.68 |
| Sample 2 | 76,46 | 86.30 |
| Sample 3 | 77,95 | 92.61 |
| Sample 4 | 78,85 | 91.24 |
| Sample 5 | 79,60 | 89.92 |
| **Average BFE result** | **78,14** | **90.15** |
| **Mean Particle Size (MPS)** | **1,81** | **3.06** |

## Claims

1. A method of testing the bacterial filtering efficiency of a fabric, the method including the steps of preparing a solution including bacteria and peptone water comprising NaCl, feeding said solution including bacteria to a nebulizer, generating an aerosol of said bacterial solution and flowing said solution through a cascade impactor to provide a plurality of bacteria colonies in a plurality of plates present in a plurality of stages of said cascade impactor, wherein said cascade impactor is an Andersen six-stages cascade impactor, wherein said fabric sample is placed between the first stage and the inlet cone of said cascade impactor, and wherein aerosol particles having a Mean Particle Size, MPS, in the range from 2.7 $\mu$m to 3.3 $\mu$m are obtained, **characterized in that** the concentration of NaCl in the peptone water used to prepare said bacterial solution is in the range of 30g/L to 150g/L and **in that** the temperature of said cascade impactor is in the range of -15°C to 15°C, whereby the aerosol particles having Mean Particle Size, MPS, in the range from 2.7 $\mu$m to 3.3 $\mu$m are obtained.

2. A method according to claim 1, wherein said method is carried out according to EN 14683:2019+AC:2019 E modified to provide an NaCl concentration of the peptone water and a refrigeration of the cascade impactor as recited in claim 1.

3. A method according to claim 1 or 2, wherein said NaCl concentration is in the range of 60g/L to 150g/L, preferably in the range of 60g/L to 120g/L, more preferably of 100g/L.

4. A method according to any previous claim, wherein the temperature of the cascade impactor is in the range of -4°C to 15°C, preferably in the range of 0°C to 12°C.

5. A method according to claim 1, wherein said nebulizer is selected from a jet nebulizer, an ultrasonic nebulizer, and a mesh nebulizer.

6. A method according to any previous claim 1 to 5, wherein said bacteria is selected from the group consisting of *Mycobacterium tuberculosis, Streptococcus pneumoniae, Legionella pneumophila, Staphylococcus aureus, Bacillus subtilis, and Escherichia coli,* preferably is *Staphylococcus aureus,* more preferably is *Staphylococcus aureus ATCC 6538,* and wherein, when said method is carried out according to EN 14683:2019+AC:2019 E, said EN 14683:2019+AC:2019 E is modified to test bacteria selected from the group consisting of *Mycobacterium tuberculosis, Streptococcus pneumoniae, Legionella pneumophila, Bacillus subtilis,* and *Escherichia coli.*

## Patentansprüche

1. Verfahren zum Testen der bakteriellen Filtereffizienz eines Gewebes, wobei das Verfahren die Schritte umfasst: Vorbereiten einer Lösung, die Bakterien und Peptonwasser mit NaCl enthält, Zuführen der bakterienhaltigen Lösung zu einem Vernebler, Erzeugen eines Aerosols aus der bakteriellen Lösung und Leiten der Lösung durch einen Kaskadenimpaktor, um eine Vielzahl von Bakterienkolonien auf der Vielzahl von Platten in einer Vielzahl von Stufen des Kaskadenimpaktors bereitzustellen, wobei der Kaskadenimpaktor ein Andersen-Kaskadenimpaktor mit sechs Stufen ist, wobei die Gewebeprobe zwischen der ersten Stufe und dem Einlasskegel des Kaskadenimpaktors positioniert wird und wobei Aerosolteilchen mit einer mittleren Teilchengröße, MPS, im Bereich von 2,7 $\mu$m bis 3,3 $\mu$m erhalten werden,

**dadurch gekennzeichnet, dass** die NaCl-Konzentration im zur Herstellung der bakteriellen Lösung verwendeten Peptonwasser im Bereich von 30 g/L bis 150 g/L liegt und dass die Temperatur des Kaskadenimpaktors im Bereich von -15 °C bis 15 °C liegt, worduch die Aerosolteilchen mit einer mittleren Teilchengröße, MPS, im Bereich von 2,7 μm bis 3,3 μm erhalten werden.

2. Verfahren nach Anspruch 1, wobei das Verfahren gemäß EN 14683:2019+AC:2019 E, modifiziert, um eine NaCl-Konzentration des Peptonwassers und eine Kühlung des Kaskadenimpaktors gemäß Anspruch 1 bereitzustellen, durchgeführt wird.

3. Verfahren nach Anspruch 1 oder 2, wobei die NaCl-Konzentration im Bereich von 60 g/L bis 150 g/L, vorzugsweise im Bereich von 60 g/L bis 120 g/L, besonders bevorzugt bei 100 g/L, liegt.

4. Verfahren nach einem der vorhergehenden Ansprüche, wobei die Temperatur des Kaskadenimpaktors im Bereich von -4 °C bis 15 °C liegt, vorzugsweise im Bereich von 0 °C bis 12 °C, liegt.

5. Verfahren nach Anspruch 1, wobei der Vernebler aus einem Jetvernebler, einem Ultraschallvernebler und einem Mesh-Vernebler ausgewählt ist.

6. Verfahren nach einem der Ansprüche 1 bis 5, wobei die genannten Bakterien aus der Gruppe ausgewählt sind, bestehend aus *Mycobacterium tuberculosis, Streptococcus pneumoniae, Legionella pneumophila, Staphylococcus aureus, Bacillus subtilis* und *Escherichia coli,* vorzugsweise *Staphylococcus aureus,* besonders bevorzugt *Staphylococcus aureus* ATCC 6538, und wobei, wenn das Verfahren gemäß EN 14683:2019+AC:2019 E durchgeführt wird, dieses EN 14683:2019+AC:2019 E modifiziert ist, um Bakterien, die aus der Gruppe, bestehend aus *Mycobacterium tuberculosis, Streptococcus pneumoniae, Legionella pneumophila, Bacillus subtilis* und *Escherichia coli* ausgewählt sind, zu testen.

## Revendications

1. Procédé d'essai de l'efficacité de filtration bactérienne d'un tissu, le procédé comprenant les étapes consistant à : préparer une solution comprenant des bactéries et de l'eau de peptone comprenant du NaCl, introduire ladite solution comprenant des bactéries dans un nébuliseur, générer un aérosol de ladite solution bactérienne et faire écouler ladite solution à travers un impacteur en cascade afin de fournir une pluralité de colonies de bactéries dans une pluralité de plaques présentes dans une pluralité d'étages dudit impacteur en cascade, dans lequel ledit impacteur en cascade est un impacteur en cascade Andersen à six étages, dans lequel ledit échantillon de tissu est placé entre le premier étage et le cône d'entrée dudit impacteur en cascade, et dans lequel des particules d'aérosol ayant une taille moyenne de particule, MPS, comprise entre 2.7 μm à 3,3 μm sont obtenues,
**caractérisé en ce que** la concentration de NaCl dans l'eau de peptone utilisée pour préparer ladite solution bactérienne est comprise entre 30g/L et 150g/L et que la température dudit impacteur en cascade est comprise entre -15°C et 15°C, ce qui permet d'obtenir des particules d'aérosol ayant une taille moyenne de particules, MPS comprise entre 2,7 μm et 3,3 μm.

2. Procédé selon la revendication 1, dans lequel ledit procédé est réalisé selon la norme EN 14683:2019+AC:2019 E modifiée pour obtenir une concentration en NaCl de l'eau de peptone et une réfrigération de l'impacteur en cascade comme indiqué dans la revendication 1.

3. Procédé selon la revendication 1 ou 2, dans lequel ladite concentration de NaCl est comprise entre 60g/L et 150g/L, de préférence entre 60g/L et 120g/L, plus préférentiellement de 100g/L.

4. Procédé selon l'une quelconque des revendications précédentes, dans lequel la température de l'impacteur en cascade est comprise entre -4°C et 15°C, de préférence entre 0°C et 12°C.

5. Procédé selon la revendication 1, dans lequel le nébuliseur est choisi parmi un nébuliseur à jet, un nébuliseur à ultrasons et un nébuliseur à mailles.

6. Procédé selon l'une quelconque des revendications précédentes 1 à 5, dans lequel ladite bactérie est choisie dans le groupe constitué de *Mycobacterium tuberculosis, Streptococcus pneumoniae, Legionella pneumophila, Staphylococcus aureus, Bacillus subtilis,* et *Escherichia coli,* de préférence est *Staphylococcus aureus,* plus préférentiellement est *Staphylococcus aureus ATCC* 6538, et dans lequel, lorsque ledit procédé est mis en œuvre conformément à la norme EN 14683 :2019+AC:2019 E, ladite norme EN 14683:2019+AC:2019 E est modifiée pour tester des bactéries choisies dans le groupe constitué de *Mycobacterium tuberculosis, Streptococcus pneumoniae, Legionella pneumophila, Bacillus subtilis* et *Escherichia coli.*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ARIEL A. ANDERSEN**. NEW SAMPLER FOR THE COLLECTION, SIZING, AND ENUMERATION OF VIABLE AIRBORNE PARTICLES. *J Bacteriol.*, November 1958, vol. 76 (5), 471-484